# EUROPEAN PATENT APPLICATION

(11) **EP 3 782 985 A1**
(43) Date of publication of application: **24.02.2021**
(21) Application number: 19192223.6
(22) Date of filing: 19.08.2019
(51) Int. Cl.: C07D 213/803, C07D 401/04

(54) **PROCESS FOR MANUFACTURING 5-METHOXYMETHYLPYRIDINE-2,3-DICARBOXYLIC ACID DERIVATIVES**

(71) Applicant: BASF Agrochemical Products B.V., 6835 EA Arnhem (NL)
(72) Inventor: ROSENKRANS, Keith W, Palmyra, MO 63461 (US); PHILLIPS, Ryan Michael, Palmyra, MO 63461 (US); CORTES, David A, Palmyra, MO 63461 (US)
(74) Representative: BASF IP Association

(57) **Abstract**

An improved process for manufacturing a compound of formula (I), 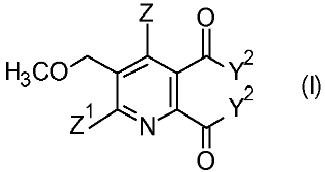 comprising steps (ii) and (iii):
(ii) reacting a mixture of compounds of formulae (III.1), (III.2) and (III.3), 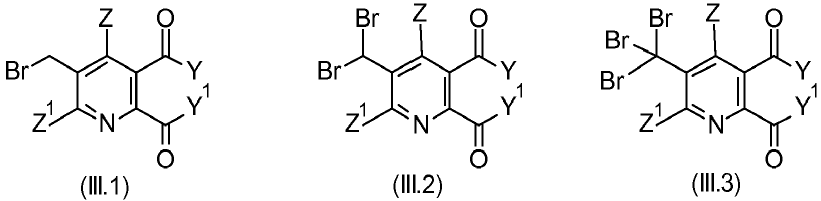 with phosphorous acid (HPO(OH)₂) or a phosphite selected from dimethyl phosphite and diethyl phosphite, and trimethylamine in a solvent at a temperature range of 0°C to 100°C, to obtain a compound of formula (II); and 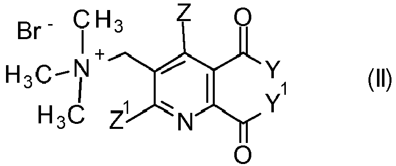 (iii) reacting the compound of formula (II) in methanol, toluene or a methanol/toluene mixture with a base selected from MOCH₃ and MOH (if the solvent is methanol), where M is as defined in formula (I), to obtain the compound of formula (I).

## Description

The invention relates to a process for manufacturing 5-methoxymethylpyridine-2,3-dicarboxylic acid [MMPDC] derivatives and further conversion of these compounds to herbicidal 5-substituted-2-(2-imidazolin-2-yl)nicotinic acids, such as imazamox.

Derivatives of 2-(2-imidazolin-2-yl) nicotinic acids, like imazamox (2-[(RS)-4-isopropyl-4-methyl-5-oxo-2-imidazolin-2-yl]-5-methoxymethylnicotinic acid), 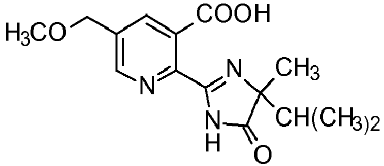 are useful herbicides, which act as inhibitors of the enzyme acetolactate synthase (ALS) and can be used in pre- and post-emergence applications.

Various processes for the synthesis of these compounds are known from the literature, see e.g. EP-A 0 322 616, EP-A 0 747 360, EP-A 0 933 362 or Q. Bi et al, Modern Agrochemicals 6(2)(2007) 10-14.

Although synthesis on an industrial scale is carried out by these methods, there is still room for improvement, specifically in view of economic and ecological aspects, such as overall yield improvement or the reduction of chemical waste streams.

EP-A 0 548 532 discloses the preparation of 5,6-disubstituted-3-pyridylmethyl ammonium halide compounds by halogenation of the respective 5,6-disubstituted-3-methylpyridines and subsequent reaction with a trialkylamine or a cyclic unsaturated or saturated amine.

Halogenating agents proposed in EP-A 0 548 532 include N-bromosuccinimide, 1,3-dibromo-5,5-dimethylhydantoin, bromine, chlorine, t-butylhypochlorite, sulfuryl chloride, sulfuryl bromide, N-chlorosuccinimide and the like; however, all examples are carried out with either N-bromosuccinimide or 1,3-dibromo-5,5-dimethylhydantoin.

WO2010/055139 discloses that the bromination of 5,6-disubstituted-3-methylpyridines and further reaction with an amine can be significantly improved by using specific solvents and bromine as bromination agent in a two-phase system with water.
However, the reaction yields by-products in significant amounts, in particular di- and tribrominated compounds, which negatively impact process management regarding yield and waste disposal:

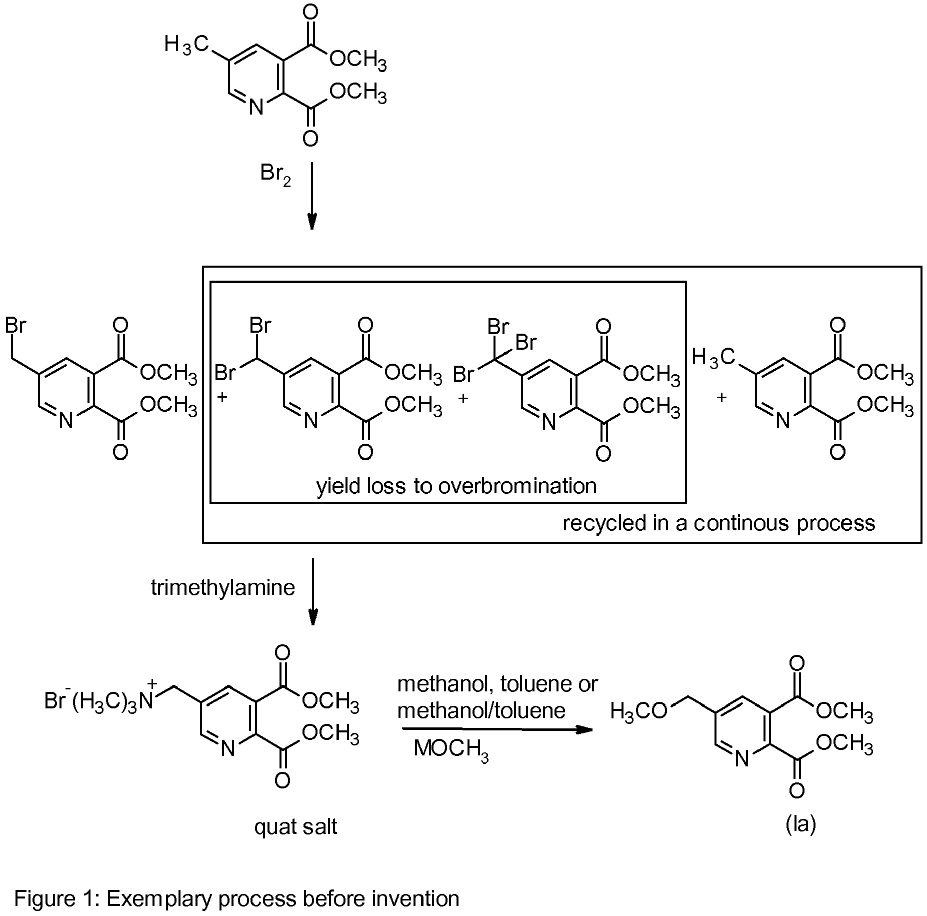

The reduction of organic halides with diethyl phosphonate and triethylamine is described by Hirao et. al. in Bull. Chem. Soc. Japan, 1983, 56, 1881-1882.

Starrett at al. disclose in US2009/011858 bromination of a substituted toluene and in situ reduction of a dibrominated product with diethyl phosphite/diisopropylamine to afford the mono-bromide.

In US 8,962,848, Miyazaki et al. describe the reaction of a 2-substituted amino-6-methylpyridine derivative and a brominating agent within an organic solvent, and reaction of the obtained product with a phosphite ester and a base within an organic solvent to obtain a monobrominated compound.

In the process for preparing methoxymethylpyridine dicarboxylate according to WO2018/091964, dialkyl-3-methylpyridine-5, 6-dicarboxylate is reacted with potassium peroxymonosulfate and a halogen metal salt to obtain a mixture comprising polybrominated compounds, which are further reacted with an amine, optionally in the presence of diethylphosphite to convert di- and trihalogenated products to the monohalogenated product.

One task of the present invention is to provide a further improved process for producing 5-methoxymethylpyridine-2,3-dicarboxylic acid [MMPDC] derivatives and conversion of these compounds to herbicidal 5-substituted-2-(2-imidazolin-2-yl)nicotinic acids, or derivatives thereof.

Accordingly, in one aspect of the invention there is provided a process for manufacturing 5-methoxymethylpyridine-2,3-dicarboxylic acid derivatives, 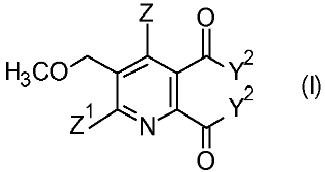 where
- Z: is hydrogen or halogen;
- Z¹: is hydrogen, halogen, cyano or nitro;
- Y²: is OCH₃ or OM; and
- M: is an alkali metal;
comprising steps (ii) and (iii):
(ii) reacting a mixture of compounds of formulae (111.1), (III.2) and (III.3), 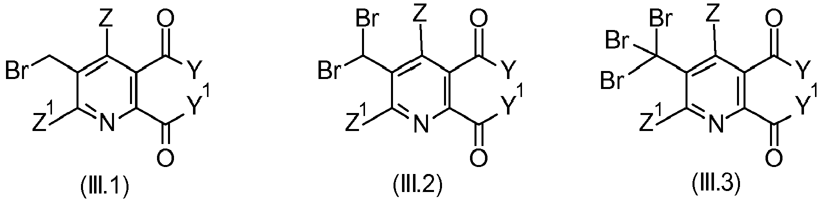 where
   - Z: is hydrogen or halogen;
   - Z¹: is hydrogen, halogen, cyano or nitro;
   - Y, Y¹: are each independently OR¹, NR¹R², or when taken together YY¹ is -O-,-S- or -NR³-;
   - R¹, R²: are each independently hydrogen, C₁-C₄ alkyl optionally substituted with C₁-C₄ alkoxy, or phenyl optionally substituted with one to three C₁-C₄ alkyl groups, C₁-C₄ alkoxy groups or halogen atoms;
   - R³: is hydrogen or C₁-C₄ alkyl;
   with phosphorous acid (HPO(OH)₂) or a phosphite selected from dimethyl phosphite and diethyl phosphite, and trimethylamine in a solvent at a temperature range of 0°C to 100°C, to obtain a compound of formula (II) 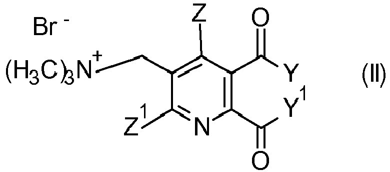 where Z, Z¹, Y, Y¹ are as defined in formulae (III.1), (III.2) and (III.3); and
(iii) reacting the compound of formula (II) in methanol, toluene or a methanol/toluene mixture with a base selected from MOCH₃ and MOH (if the solvent is methanol), where M is as defined in formula (I), to obtain the compound of formula (I).

In a further aspect of the invention, there is provided a process for manufacturing 5-methoxymethylpyridine-2,3-dicarboxylic acid derivatives, where the mixture of compounds of formulae (III.1), (III.2) and (III.3) used in step (ii) is prepared in step (i) comprising:
(i) reacting a compound of formula (IV), 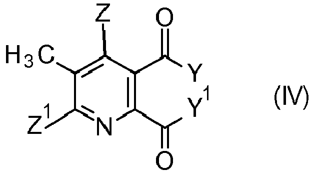 where
   - Z: is hydrogen or halogen;
   - Z¹: is hydrogen, halogen, cyano or nitro;
   - Y, Y¹: are each independently OR¹, NR¹R², or when taken together YY¹ is -O-,-S- or -NR³-;
   - R¹, R²: are each independently hydrogen, C₁-C₄ alkyl optionally substituted with C₁-C₄ alkoxy, or phenyl optionally substituted with one to three C₁-C₄ alkyl groups, C₁-C₄ alkoxy groups or halogen atoms;
   - R³: is hydrogen or C₁-C₄ alkyl;
   with bromine in the presence of a radical initiator in a solvent mixture comprising an aqueous phase and an organic phase, where the organic phase comprises a solvent selected from 1,2-dichloroethane, chlorobenzene, 1,2-dichlorobenzene, 1,3-dichlorobenzene, 1,4-dichlorobenzene and tetrachloromethane, and where the pH-value of the aqueous phase is from 1 to < 8.
   In a further aspect of the invention there is provided a process for preparing a herbicidal imidazolinone compound of formula (V), 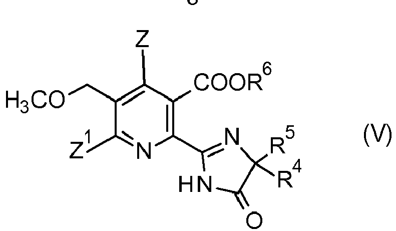 where
   - Z: is hydrogen or halogen;
   - Z¹: is hydrogen, halogen, cyano or nitro;
   - R⁴: is C₁-C₄ alkyl;
   - R⁵: is C₁-C₄ alkyl, C₃-C₆ cycloalkyl, or R⁴ and R⁵, when taken together with the atom to which they are attached, represent a C₃-C₆ cycloalkyl group optionally substituted with methyl; and
   - R⁶: is hydrogen;
   comprising the steps of:
   preparing a compound having the formula (I) comprising steps (ii) and (iii) or (i), (ii) and (iii) as described above; and
(v) converting the compound of formula (I) into the herbicidal compound having formula (V).

The process of the invention leads to higher yields, higher productivity (higher space time yields, lower fixed costs), lower raw material costs, improved solvent recovery, and a reduced waste stream (di- and tribrominated by-products are converted to the monobrominated product and can be reacted to the desired compound of formula (I)) and an improved selectivity for the compounds of formula (I).

Particularly preferred are the compounds of formula (I) where the symbols have the following meanings:
- Z and Z¹: are hydrogen.
- Y²: is OCH₃.

Also, particularly preferred are the compounds of formula (I) where the symbols have the following meanings:
- Z and Z¹: are hydrogen.
- Y²: is OM.

Accordingly, a particularly preferred compound of formula (I) is the compound of formula (Ia):

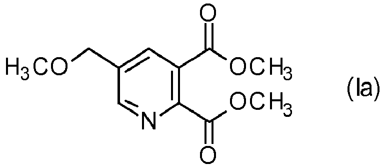

Preferred, more preferred and particularly preferred compounds of formula (II) are the ones leading to the respective compounds of formula (I), e.g. compounds of formula (II),
where
- Z: is hydrogen;
- Z¹: is hydrogen;
- Y, Y¹: are each independently OR¹;
- R¹: is hydrogen, or C₁-C₄ alkyl;
in particular the compound of formula (IIa):

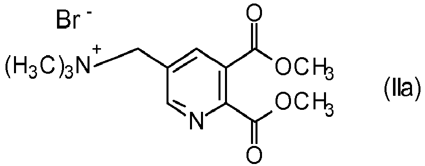

The compounds of formula (II) and their preparation are known, e.g. from EP-A 0 933 362.

Preferred, more preferred and particularly preferred compounds of formula (IV) are the ones leading to the respective compounds of formula (I), e.g. compounds of formula (IV),
where
- Z: is hydrogen;
- Z¹: is hydrogen;
- Y, Y¹: are each independently OR¹;
- R¹: is hydrogen or C₁-C₄ alkyl;
in particular the compound of formula (IVa).

Schematic representation of the inventive process starting from particularly preferred compound of formula (IVa):

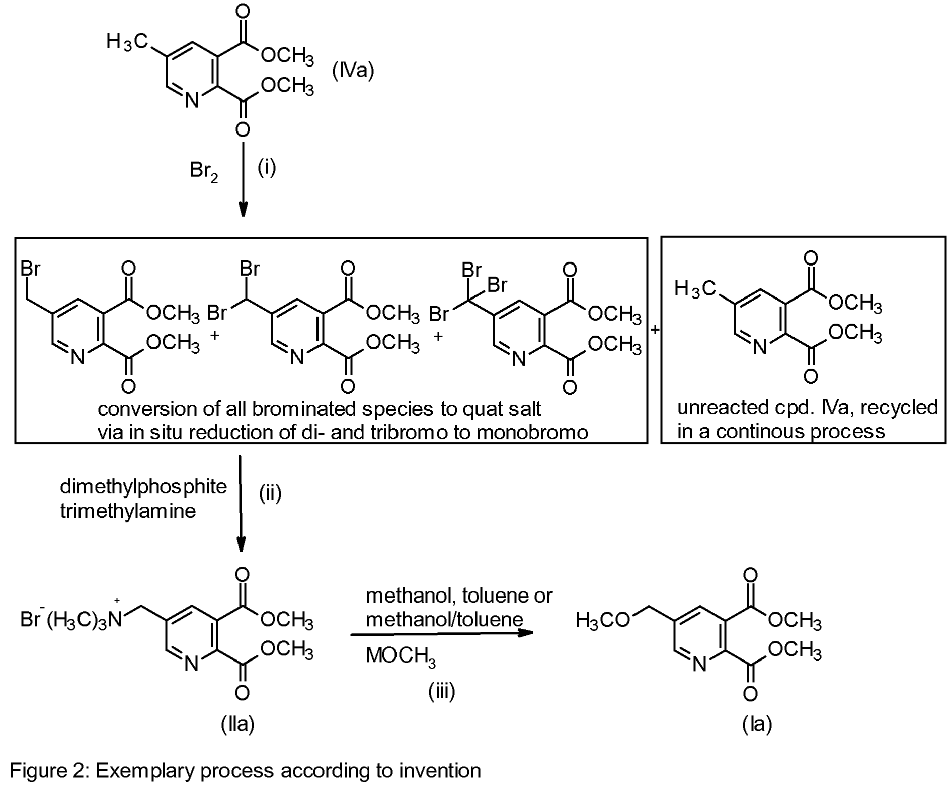

### Step (i):

The molar ratio of pyridine compound (IV) to bromine is generally in the range of 1:0.5-1.2, preferably 1:0.6-1.0, more preferably 1:0.7-0.95.

It is also possible to work with half the equivalents of bromine, and to generate bromine in the reaction mixture (i.e. in situ) from bromides (e. g. HBr, halogen metal salts such as NaBr) with an oxidation agent like H₂O₂ or potassium peroxymonosulfate.

Suitable free-radical generators for initiating the reaction are those which decompose at the selected reaction temperature. Examples of preferred initiators are free-radical generators, such as azo compounds and peroxides. It is also possible, however, to use redox systems, especially those based on hydroperoxides, such as cumene hydroperoxide.

Radical initiators suitable for use in the method of the invention include 2,2'-azobisisobutyronitrile (AIBN), 2,2'-azobis(2-methylbutanenitrile), 2,2'-azobis(2,4-dimethyl-pentanenitrile), 1,1'-azobis(cyclohexanecarbonitrile), organic and inorganic peroxides such as dilauroyl peroxide, hydrogen peroxide, *tert*-butylperoxy-pivalate, benzoyl peroxide and the like, with 2,2'-azobisisobutyronitrile, 2,2'-azobis(2-methylbutanenitrile) and dilauroyl peroxide being preferred, and with 2,2'-azobisisobutyronitrile and 2,2'-azobis(2-methylbutanenitrile) being particularly preferred.

The molar ratio of initiator to bromine is preferably in the range of 0.04-0.15:1, more preferably 0.06-0.10:1.

The organic solvent is selected from the group consisting of 1,2-dichloroethane, chlorobenzene 1,2-dichlorobenzene, 1,3-dichlorobenzene, 1,4-dichlorobenzene, dichloromethane, and tetrachloromethane, preferably 1,2-dichloroethane and chlorobenzene. Mixtures, in particular of the dichlorobenzenes, are also possible. 1,2-dichloroethane is particularly preferred.

The amount of organic solvent may vary to a large extent. Preferably 900 g to 2000 g, more preferably 1000 g to 1300 g, organic solvent per mol of compound (IV) are employed.

The reaction mixture comprises an organic phase and an aqueous phase. The amount of the aqueous phase may vary to a large extent. Preferably 140 g to 500 g, more preferably 140 g to 300 g, particularly 150 g to 200 g of water per mol of the compound of formula (IV) are employed.

During the course of the reaction the pH-value of the aqueous phase is kept in the range of from 1 to < 8, preferably of from 1 to 7, more preferably of from 3 to 7. Control of the pH-value can be achieved by adding a suitable base, preferably an inorganic base such as a hydroxide of an alkali metal, e.g. NaOH. Aqueous NaOH is a preferred base, particularly in diluted form (e.g. containing 5-20 wt.-% NaOH).

To achieve the desired control of the pH-value the base may be added continuously over the course of the reaction, or the pH-value is checked continuously, and base is added by a connected automated dosage device.

In one preferred embodiment step (i) of the reaction is carried out by dissolving the compound (IV) in the organic phase and water adding to form the aqueous phase.

The initiator is added as pure compound or in solution, at room temperature (generally a temperature in the range of from 22 to 25°C) or at reaction temperature after heating. Depending on the initiator decomposition temperature, a part or even the full amount of the initiator has to be added before the start of the bromine dosage. The amount of starter that has to be added during the bromine addition is also depending on the decomposition temperature. A minimum concentration of free radicals should be always available during the bromination reaction.

For 2,2'-azobis(2-methylbutanenitrile), a solution with initiator in an organic solvent is added. Slow addition of bromine as well as of the base to control the pH-value can be started at the same time or some time later. It is preferred to start the bromine/base dosage later in order to have a sufficient amount of free radicals in the mixture when the bromination reaction starts. After completion of the reaction the mixture is cooled, and the phases are separated. The reaction is generally carried out at a temperature of about 50°C to about 120°C, preferably about 60°C to about 90°C.

The reaction may be carried out under atmospheric pressure or under excess pressure of up to 6 bar. Atmospheric pressure is preferred.

The reaction time (for step (i)) differs with the reaction parameters but is generally between 1 h and 24 h.

In order to improve overall yield and to enhance selectivity of the reaction, i.e. to reduce formation of the undesired dibromo and tribromo by-products, it is preferred to carry out the reaction only up to a conversion of 5 to 60% (based on the amount of compound (IV)), preferably 20 to 60 %, more preferably 30 to 55%. In one preferred embodiment the reaction is carried out up to a conversion of about 50% (based on compound (IV)). The degree of conversion may be checked by standard methods known to those skilled in the art, e.g. by HPLC analysis.
When the desired degree of conversion is reached the reaction is stopped and the phases are separated.

The organic phase, containing the products of step (i), compounds (III.1), (III.2), (III.3), and unreacted starting material (IV), may be extracted with water to remove water soluble impurities like acids and bromide. Product ((III.1), (III.2), (III.3)) can be isolated by known procedures, it is preferred, however, to use the organic phase without further work-up for the reaction in step (ii).

It is also possible to extract the aqueous phase with the organic solvent and to combine the organic phases to increase the yield of compounds (III.1), (III.2), (III.3).

### Step (ii):

In step (ii), the reaction mixture obtained in step (i) or a mixture of compounds of formulae (111.1), (III.2) and (III.3) is reacted with phosphorous acid (HPO(OH)₂) or a phosphite selected from dimethyl phosphite and diethyl phosphite, and the tertiary amine trimethylamine (N(CH₃)₃, TMA) in a solvent to obtain the compound of formula (II).

In the context of the present invention, dimethyl phosphite (HPO(OCH₃)₂) is particularly preferred.

Generally, an excess of phosphorous acid or the phosphite is used. Typically 2 to 4, preferably 2.0 to 3.2 equivalents of phosphorous acid or the phosphite per equivalent of the combined moles of compounds of formula (III.2) and (III.3) are employed.

Generally, an excess of the amine, trimethylamine (TMA), is used. Typically, 1.05 to 2, preferably 1.05 to 1.5, more preferably 1.05 to 1.4 equivalents of amine per equivalent of the combined moles of compounds of formula (III.1), (III.2) and (III.3) are employed.

Generally, the phosphorous acid or the phosphite is added to the solution comprising compounds (III.1), (III.2) and (III.3), then TMA, optionally dissolved in a solvent, is slowly added to the solution comprising compounds (III.1), (III.2) and (III.3) and phosphorous acid or the phosphite, whereupon the salt (II) forms and precipitates. As the preferred amine is TMA, which is gaseous at room temperature, it is preferred to work in a closed vessel and charge the gaseous amine or the liquefied amine under pressure to the reaction solution.

Generally, the solvent in step (ii) is an inert organic, non-polar, halogenated solvent. Preferably, the organic solvent is selected from the group consisting of 1,2-dichloroethane, chlorobenzene 1,2-dichlorobenzene, 1,3-dichlorobenzene, 1,4-dichlorobenzene, dichloromethane, and tetrachloromethane, preferably 1,2-dichloroethane and chlorobenzene. Mixtures, in particular of the dichlorobenzenes, are also possible. 1,2-dichloroethane is particularly preferred.

In a preferred embodiment the solvent in step (ii) is the organic phase with the product from step (i) plus additional solvent, e.g. 1,2-dichloroethane.

Generally, step (ii) is carried out at a temperature of about 0°C to 100°C preferably at a temperature of about 0°C to 70°C, more preferably at a temperature of about 5°C to 70°C, particularly preferred at a temperature of 5°C to 55°C. The reaction can be carried out at ambient pressure or at elevated pressure. In a preferred embodiment, the reaction is carried out in a closed vessel at the pressure of the solvent and/or amine building up at the reaction temperature.

Work up of the reaction mixture and isolation of the ammonium compound (II) can be carried out by conventional methods, e.g. compound (II) can be filtered off.

In a preferred embodiment water is added to the reaction mixture to dissolve the product, compound (II), and aqueous phase and organic phase are separated. The water phase may be further extracted with organic solvent in order to increase the purity of the compound (II), and, provided step (i) was performed, to increase the yield of recovered starting material (IV) in the organic phase. The amount of water must be sufficient for an aqueous phase to form and is preferably chosen to form a 20 to 45% by weight solution of compound (II) in the aqueous phase.

Ammonium compound (II) can be isolated from the aqueous phase by known methods. In a preferred embodiment compound (II) is not isolated and the aqueous phase obtained from step (ii) is used in subsequent reactions without further workup. In a further preferred embodiment the aqueous phase is mixed with a solvent that forms an azeotrope with water, e.g. toluene, and water is removed by azeotropic distillation. The resulting suspension of compound (II) can be used for further reactions.

In a further preferred embodiment of the invention, after separation of compound (II), the organic phase from step (ii) containing up to 80% of starting material (IV) (based on the original amount used in step (i)) is recovered and recycled in the reaction process of step (i). Preferably, further starting material (IV) is added to compensate for the amount converted in previous step (i). Thus, in principle, the organic phase of step (i) can be recycled any amount of times, however, due to an accumulation of by-products, up to 20, preferably up to 10 cycles are generally feasible.

In a preferred embodiment of the cyclic reaction process no additional starting material (IV) is added in the last cycle to improve the overall yield and conversion rate.

In a further embodiment of the cyclic reaction process a certain amount of the organic phase, preferably about 5 to 20% by weight, are removed to reduce or suppress accumulation of by-products in the organic phase. In this embodiment of the invention there is virtually no limit to the number of cycles in which the organic phase can be used.
The compounds of formula (II) are valuable intermediates in organic synthesis. They are especially useful for conversion to methoxymethyl compounds (I) and further to herbicidal imidazolinone compounds (V).

### Step (iii):

In one aspect of the invention, there is provided a process for producing compounds of formula (I) comprising the steps of:
(ii) preparing a 5,6-disubstituted-3-pyridylmethyl ammonium bromide of the formula (II) as described above, and
(iii) reacting the compound of formula (II) in methanol, toluene or a methanol/toluene mixture with a base selected from MOCH₃ and MOH (if the solvent is methanol), where M is as defined in formula (I).

In another aspect of the invention, there is provided a process for producing compounds of formula (I) comprising the steps of:
(i)/(ii) preparing a 5,6-disubstituted-3-pyridylmethyl ammonium bromide of the formula (II) as described above, and
(iii) reacting the compound of formula (II) in methanol, toluene or a methanol/toluene mixture with a base selected from MOCH₃ and MOH (if the solvent is methanol), where M is as defined in formula (I).

The base MOH can be used in form of an aqueous solution. Aqueous bases suitable for use in this embodiment of the invention include aqueous sodium hydroxide solution, aqueous potassium hydroxide solution and the like.

Organic solvents that may be used in the method of the invention include acetonitrile, tetrahydrofuran, aromatic hydrocarbons, methanol and the like. The preferred inert organic solvent is methanol, toluene, or a mixture thereof.

Suitable reaction temperatures are about 120° to 180°C, preferably about 120° to 150°C. Reaction pressures would be those pressures which normally accompany heating a solvent in a closed reaction system to a temperature range above its boiling point.

Derivatives of compounds of formula (I), where Y² is OH, may be obtained by acidification of the respective dicarboxylates.

Acids that may be used in the method of the invention include mineral acids such as sulfuric acid, hydrochloric acid and the like.

In one preferred embodiment, where Y² is OCH₃ or OH, step (iii) is carried out as disclosed in WO2010/066669, i.e. by reaction of the respective compound of formula (II) in a methanol/H₂O mixture, comprising at least 20% by weight H₂O (based on the sum of water and bromide (II)), with a base comprising MOCH₃ and/or MOH, where M is an alkali metal, under pressure in a closed vessel at a temperature from 75 to 110°C. Preferably the amount of water is from about 25 to about 75%, more preferably 30 to 70%, in particular from 15 about 40 to about 50%. The remainder of the solvent mixture is methanol and up to about 50%, preferably up to about 20% of further solvents, preferably selected from toluene, chlorobenzene and ethanol.
In a preferred embodiment, bromide (II), containing from about 25 to 75 % by weight water (based on the sum of water and bromide (II)), is taken up in methanol, aqueous NaOH is slowly added at a temperature in the range of from about 25 to 40°C, followed by NaOCH₃ in methanol which is slowly added at a temperature in the range of from about 40 to 50°C. The reaction mixture is then heated to the reaction temperature (typically 80 to 100°C) in a pressure reactor, which is closed, whereupon pressure builds up as the reaction temperature is reached.
After completion of the reaction the mixture is cooled down and can be worked up according to known procedures, e.g. by cooling, treatment with an acid, such as sulfuric acid, until compound (I) precipitates and can be filtered off.

In another preferred embodiment, where Y² is OM, step (iii) is carried out as disclosed in EP-A 0 747 360, i.e. by reaction of the respective compound of formula (II) in methanol with a base.

Bases suitable for use in this embodiment of invention are alkali metal hydrides, hydroxides, carbonates or C₁-C₄ alkoxides. Alkali metals such as sodium or potassium are preferred. Particularly preferred bases are sodium or potassium hydroxide.

In a further preferred embodiment, where Y² is OCH₃ or OH, step (iii) is carried out as disclosed in EP-A 0 548 532, i.e. by reacting compound (II) with MOCH₃, where M is an alkali metal such as Na or K, in the presence of an organic solvent preferably at a temperature range of 0°C to 110°C to form a first mixture further reacting said first mixture with at least 2.0 molar equivalents of an aqueous base preferably at a temperature range of about 20°C to 120°C to form a second mixture and adjusting the pH of said second mixture to a value below 2.5 with an acid to form acid compounds of formula (I).

In another preferred embodiment, where Y² is OCH₃, step (iii) is carried out as disclosed in EP-A 0 548 532, and the compound of formula (I) is reacted with MOCH₃, where M is Na or K, in an inert solvent, preferably methanol.

Further conversion of compound (I) to herbicidal imidazolinones (V) can be achieved by methods known in the art.

Methods that may be used to create the imidazolinone herbicides are illustrated in the book "The Imidazolinone Herbicides" edited by D. L. Shaner and S. L. O'Connor, published 1991 by CRC Press, Boca Raton, Florida with particular reference to Chapter 2 entitled "Synthesis of The Imidazolinone Herbicides", pages 8-14 and the references cited therein. The following patent literature references also illustrate the methods that may be used to convert the pyridine diacids, esters and salts to the imidazolinone final products:
US Patents Nos. 5371229; 5250694; 5276157; 5110930; 5122608; 5206368; 4925944; 4921961; 4959476; 5103009; 4816588; 4757146; 4798619; 4766218; 5001254; 5021078; 4723011; 4709036; 4658030; 4608079; 4719303; 4562257; 4518780; 4474962; 4623726; 4750978; 4638068; 4439607; 4459408; 4459409; 4460776; 4125727 and 4758667, and EP-A-0 041 623.

In one embodiment the conversion of compound (I) to a herbicidal imidazolinone (V) is carried out in analogy to the method disclosed in EP-A 0 233 150 or B.I. Quang et al., Modem Agrochemicals 6 (2007) p. 14.

In this embodiment a herbicidal imidazolinone compound (V) is prepared by
preparing a compound having the formula (I) comprising steps (ii) and (iii) or (i), (ii) and (iii) as outlined above; and
(iv) reacting compound (I) in the presence of a strong base with an 2-aminoalkane carboxamide of formula (VI),

H₂N-CR⁴R⁵-CONH₂ (VI),

where R⁴ and R⁵ are as in formula (V),
and adjusting the pH to give a compound of formula (V).

The reaction is carried out in an inert solvent, e.g. aromatic hydrocarbons and halogenated hydrocarbons, such as toluene, alcohols, such as methanol or tert-butanol. Water immiscible solvents are preferred. Suitable strong bases are alkali metal alcoholates and alkali metal hydroxides, such as NaOCH₃ or KO-tert-C₄H₉. The reaction is carried out in the range of from room temperature (generally 22°C) to reflux temperature of the reaction mixture, preferably 50 to 90°C.

In further embodiments of the invention conversion of compound (I) to a herbicidal imidazolinone (V) is carried out in analogy to the methods described in US 5,334,576.

According to these embodiments compound (I) is first converted to the respective anhydride by known methods, such as preparation of the corresponding dicarboxylic acid and subsequent reaction with acetic anhydride US 5,334,576 (examples 5 and 6).

In one embodiment compound (V) is prepared by
preparing a compound having the formula (I) comprising steps (ii) and (iii) or (i), (ii) and (iii) as outlined above;
(iv-1) conversion of compound (I) to the anhydride (VII), 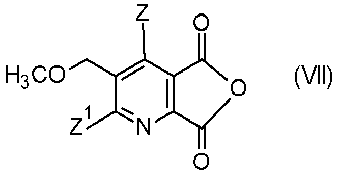
(iv-2) reacting anhydride (VII) with an 2-aminoalkane carboxamide of formula (VI),

   H₂N-CR⁴R⁵-CONH₂ (VI),

   to yield amide (VIII), 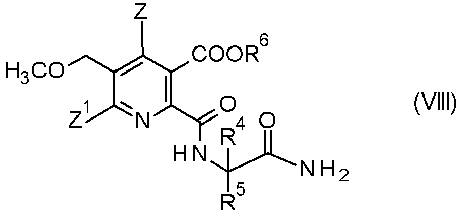 and
(iv-3) condensation of amide (VIII) to yield the herbicidal imidazolinone (V).

Steps (iv-2) and (iv-3) may be carried out as a one-pot reaction.

In one embodiment step, (iv-2) is carried out in analogy to the procedure disclosed in example 10 of EP-A 0 322 616. Compound (I), a substituted 2-aminoalkane carboxamide (VI) and a tertiary amine, preferably triethylamine are reacted in a polar aprotic solvent, such as acetonitrile, to yield an ammonium salt (VIII) (R⁶ = HNR₃), which can be acidified to an acid (VIII) (R⁶ = H).

Alternative procedures are disclosed in US 4,518,780 and EP-A 0 144 595. In the latter document the addition of a nitrogen base selected from pyridine, the picolines, quinoline and lutidine is disclosed to improve the regioselectivity of the reaction, i.e. to increase the amount of 2-addition product.

In one embodiment of step (iv-3) amido compound (VIII), preferably in the form of an ammonium salt (R⁶ is HNR₃), is reacted with an alkali metal methoxide, preferably NaOCH₃ in methanol in analogy to example 11 of EP 0 322 616. The resulting suspension is held at reflux until complete conversion. After cooling the mixture is acidified to obtain compound (V) either as the ammonium salt (acidification to a pH of about 4) or the free acid (acidification to pH ≤ 2).

In a further preferred embodiment, the reaction mixture from step (iv-2) is reacted with methanol (generally 2 to 100 equivalents based on (VIII)) in the presence of an aqueous base (generally 3 to 100 equivalents based on (VIII)), the base being preferably selected from MOH and MOCH₃, where M is an alkali metal, preferably Na or K, particularly Na.

The reaction is carried out at a temperature in the range of from 20 to 120°C, preferably 40 to 90°C. The reaction can be carried out at atmospheric pressure or at elevated pressure, preferably the pressure forming at the desired reaction temperature. The reaction time is generally from 1 to 8 h, preferably from 1 to 5 h.

Isolation of product (V) can be achieved by standard methods. In a preferred embodiment water is added and organic solvents are distilled off. The residue can be taken up in water and acidified, whereupon compound (V) precipitates. After filtration the crude product can be further purified, e.g. by stirring with water or recrystallization.

In a further embodiment compound (V) is prepared by
preparing a compound having the formula (I) comprising steps (ii) and (iii) or (i), (ii) and (iii) as outlined above;
(iv-1) conversion of compound (I) to the anhydride (VII), 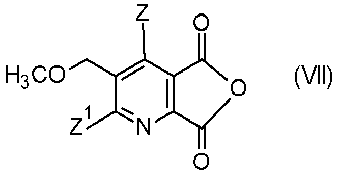
(iv-2) reacting anhydride (VIII) with aminocarbonitrile (IX),

   H₂N-CR⁴R⁵-CN (IX)

   where R⁴ and R⁵ are as in formula (V),
   to obtain amidonitrile compound (X), 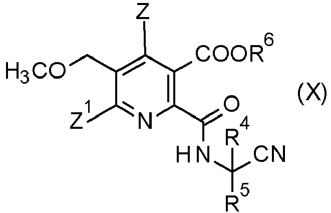 where the symbols are as in formula (V) and R⁶ is preferably H,
(iv-3) hydrolysis of the nitrile group in compound (X) to yield amide (VIII), 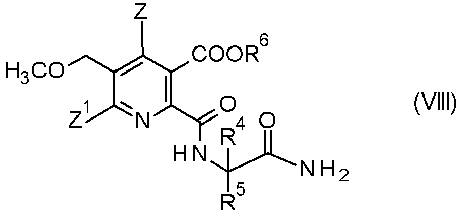 where the symbols have the same meaning as in formula (V) and R⁶ is preferably H, and
(iv-4) condensing amide (VIII) to yield the herbicidal imidazolinone (V).

Preparation of the anhydride can be carried out as described above.

Aminonitriles (IX), which are employed in step (iv-2), are commercially available or can be prepared by methods known in the art. Generally, 0.8 to 1.2 equivalents aminonitrile (IX) per equivalent of compound (I) are used, preferably 0.95 to 1.1.

The reaction is carried out in a solvent, which is preferably selected from aromatic hydrocarbons, preferably toluene, mesitylenes, chlorinated aromatic hydrocarbons, such as chlorobenzene, dichlorobenzene, chlorinated hydrocarbons, such as 1.2-dichloroethane, dichloromethane, acetic acid, and mixtures thereof.

If acetic acid is not used as the main solvent, addition of 0.5 to 4 equivalents, preferably 1 to 3 equivalents (based on compound (I)), is advantageous. Further advantageous additives that improve the selectivity of the ring-opening reaction (2 versus 3 position) are listed in EP-A 0 144 555, and comprise pyridine, 4-picoline, 2-picoline and quinoline.

The reaction is generally carried out at a temperature range of from about 40 to about 120°C, preferably of from about 60 to about 100°C. The reaction time is generally from about 1 to about 3 h.

In a preferred embodiment compound (I) is first converted to the corresponding dicarboxylic acid, reacted with acetic anhydride to obtain the anhydride (VII), which is dissolved in the solvent, brought to the reaction temperature, and aminonitrile (IX) is gradually added. After completion of the reaction and cooling, nitrile compound (X) can be isolated by standard methods.

In a preferred embodiment, however, compound (X) is not isolated but the reaction mixture is directly used in the following hydrolyzation step of the nitrile (step iv-3).
In a typical procedure a slight excess (e.g. 1.1 to 1.5 equivalents based on (IX)) of a strong mineral acid, preferably sulfuric acid (preferably in a concentration of 30 to 98%) and water (e.g. 2 to 10 equivalents) are added at a temperature which is generally in the range of about 30°C to 120°C, preferably 50°C to 90°C. The mixture is further stirred until complete conversion. The reaction time is generally from 1 to 8 h, preferably 1 to 5 h.

Workup and isolation of amide (VIII) can be achieved by standard methods, such as an aqueous solution (e.g. as its ammonium salt). In a preferred embodiment the reaction mixture is directly used in the following condensation step (iv-4).

In an alternative embodiment hydrolysis of the nitrile group is effected by reaction with aqueous NaOH/H₂O₂ as disclosed, e.g. in EP-A 0 144 595 and US 4,518,780.

Condensation of amido compound (X) to the herbicidal imidazolinone may be carried out as described above.

All of the above processes are particularly preferred for the preparation of the compound of formula (V) where Z and Z¹ are H, R⁶ is H, R⁴ is CH₃ and R⁵ is CH(CH₃)₂, i.e. imazamox.

The invention is illustrated by the following examples without limiting it thereby.

### Examples

### Example 1

### Synthesis of [(5,6-dicarboxy-3-pyridyl)methyl] trimethylammonium bromide, dimethylester (IIa)

a) Synthesis of dimethyl 5-(bromomethyl)-2,3-pyridinedicarboxylate (III.a) (50% conversion): step (i) 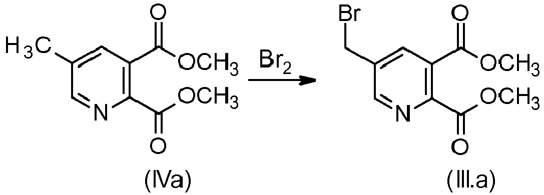 85.4 g (0.4 mol) compound (IVa) were dissolved in 443.8 g 1,2-dichloroethane (EDC) and 59.0 g water were charged and heated to 72°C (about 1-2°C below reflux). 5.8 g (0.075 mol) 2,2'-azobis(2-methylpropionitrile) (Vazo 64) in 50.7 g EDC were added over 30 min at 72°C. After 30 minutes 48.2 g (0.3 mol) bromine were added over 2 h, under pH control (pH 3-5) by dosage of about ca. 103.3 g aqueous NaOH (15%). The mixture was stirred over 1 h for reaction completion (HPLC assay). After cooling to 40°C the phases were separated.
b) Synthesis of compound (IIa): step (ii) 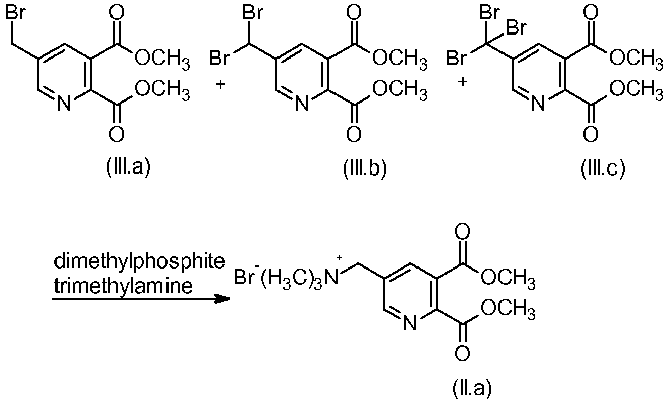 51.4 g (0.178 mol) compound (III.a) in mixture with 7.9 g (0.0216 mol) compound (III.b), and no-detected level of compound (III.c) (di- and tribromination by-products) in 590.0 g EDC (organic phase from step a, including unreacted compound (IVa) and higher brominated by-products) were charged. The mixture was heated to 30°C. 8.5 g (0.0757 mol) of dimethyl phosphite is charged, and the vessel evacuated to 200 mbar. 15.4 g (0.258 mol) trimethylamine (TMA) was added subsurface via dip tube during 2 h at 40°C (closed system). The mixture was stirred 1 additional hour (HPLC conversion check: compound (III.a) in solution < 0.1%).
   Excess TMA was distilled off together with EDC (mass: 40% of the EDC mass transferred to step 2 (149.3 g) at 50-55°C (370-250 mbar). The pH of the distillate was < 9. 105.5 g water was sprayed to the wall so that the solid is dissolved and the mixture was transferred to the next vessel. The mixture was then stirred 0.25 h, and the lower organic phase was separated at 40°C. 27.9 g EDC were added. The mixture was stirred and the lower organic phase was separated at 40°C. The back extraction was repeated (40°C) with 27.9 g EDC. The two organic back extraction phases were combined with the first organic phase and recycled to the next bromination batch (after addition of 50% fresh compound (IVa) for a further cycle.

Steps a) and b) were repeated six times. In the last cycle no compound (IVa) was added in step a) and 0.166 mol TMA were added in step b).

The overall conversion rate of compound (IVa) was 95.5 %. The yield of compound (IIa) (over 7 cycles) was 84.1 % at a purity of > 95 % (as determined by HPLC).

### Example 2

### Synthesis of [(5,6-dicarboxy-3-pyridyl)methyl] trimethylammonium bromide, dimethylester (IIa)

a) Synthesis of dimethyl 5-(bromomethyl)-2,3-pyridinedicarboxylate (III.a) (Full conversion): step (i) 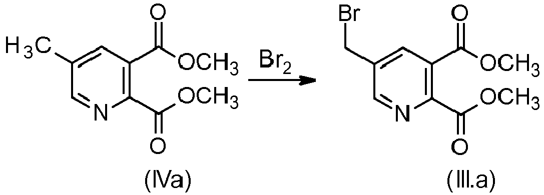 167.4 g (0.80 mol) compound (IVa) were dissolved in 887.6 g 1,2-dichloroethane (EDC) and 120.5 g water were charged and heated to 72°C (about 1-2°C below reflux). 11.4 g (0.0.0688 mol) 2,2'-azobis(2-methylpropionitrile) (Vazo 64) in 101.6 g EDC were added over 30 min at 72°C. After 30 minutes 257.0 g (1.6 mol) bromine were added over 2 h, under pH control (pH 3-5) by dosage of about ca. 582.4 g aqueous NaOH (15%). The mixture was stirred over 1 h for reaction completion (HPLC assay). After cooling to 40°C the phases were separated.
b) Synthesis of compound (IIa): step (ii) 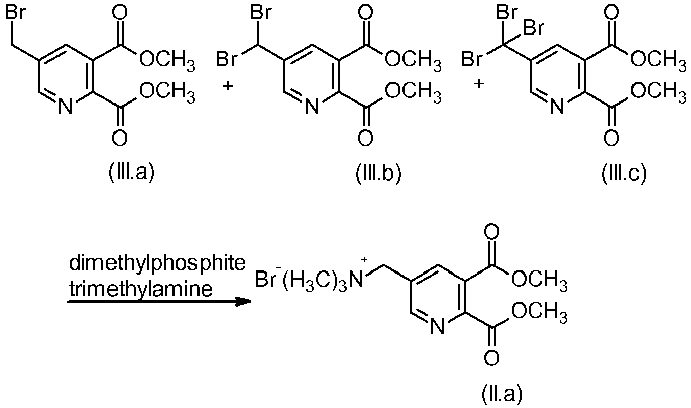 116.0 g (0.4028 mol) compound (III.a) in mixture with 117.9 g (0.3213 mol) compound (III.b), and 10.0 g (0.0225) (III.c) (di- and tribromination by-products) in 1238.0 g EDC (organic phase from step a, including unreacted compound (IVa) and higher brominated by-products) were charged. The mixture was heated to 30°C. 127.4 g (1.135 mol) of dimethyl phosphite is charged, and the vessel evacuated to 200 mbar. 92.2 g (1.544 mol) trimethylamine (TMA) was added subsurface via dip tube during 2 h at 40°C (closed system). The mixture was stirred 1 additional hour (HPLC conversion check: compound (III.a) in solution < 0.1%).
   The reaction slurry was cooled to 20°C and filtered to obtain a solid. The solid is washed with 260.0 g EDC, and vacuum dried at 50°C to give 258.6 g (0.745 mol) of isolated compound (IIa). The conversion rate of compound (IVa) was 95.9 %. The yield of compound (IIa) was 93.0 % at a purity of > 95 % (as determined by HPLC).

### Example 3

### Preparation of dimethyl 5-(methoxymethyl)-2,3-pyridinedicarboxylate (according to ex. 7 of EP-A 0 548 532): step (iii):

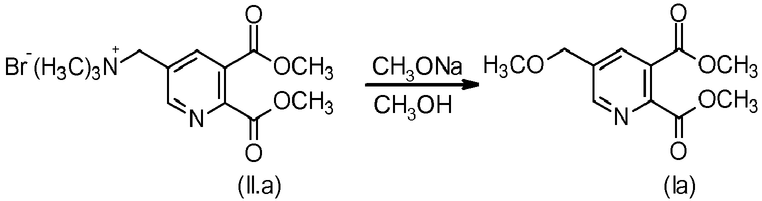

A mixture of 25% sodium methoxide in methanol (320.0 g, 1.5 mol) and [(5,6-dicarboxy-3-pyridyl)-methyl]trimethylammoniumbromide, dimethyl ester (160.0 g, 0.5 mol) in methanol (630 ml) is heated at reflux for 6 hours under nitrogen. The reaction mixture is cooled to 5°C and acetic acid (90 g) and water (200 ml) are added. Methanol is removed *in vacuo,* water is added and the mixture is extracted with methylene chloride.

### Example 4

### Preparation of 5-(methoxymethyl)-2,3-pyridinedicarboxylic acid (according to ex. 8 of EP-A 0 548 532)

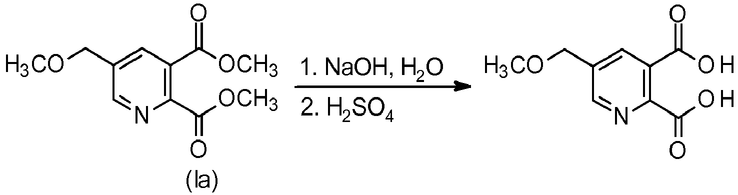

A mixture of dimethyl 5-(methoxymethyl)-2,3-pyridinedicarboxylate (60.0 g, 0.25 mol) and 50% sodium hydroxide solution (50.0 g, 0.63 mol) in water is heated at 90-110°C for 2 hours under nitrogen while distilling off methanol and water. The reaction mixture is cooled to 10°C, treated with sulfuric acid to adjust the pH to 2.0 and filtered to obtain a solid. The solid is washed with water and vacuum dried to give the title product as a white solid (44.3 g, mp 161-162°C).

### Example 5

### Preparation of 5-(methoxymethyl)-2,3-pyridinedicarboxylic acid (according to ex. 3 of EP-A 0 548 532)

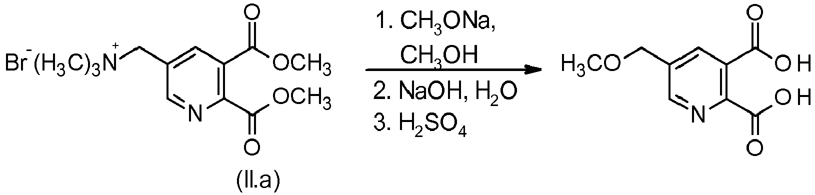

A mixture of 25% sodium methoxide in methanol (270 g, 1.25 mol) and [(5,6-dicarboxy-3-pyridyl)-methyl]trimethylammonium bromide, dimethyl ester (IIa) (347 g, 1.00 mol) in methanol (650 ml) is heated at reflux for 1 hour under nitrogen. Water (1 I) and sodium hydroxide (80.0 g, 2.0 mol) are added and the reaction mixture is distilled until the pot is 100-105°C. The reaction mixture is cooled to room temperature, treated with sulfuric acid to adjust the pH to a value from 1.5 to 2 and filtered to obtain a solid. The solid is washed with water and dried in a vacuum oven to obtain the title product as a white solid (mp 161-162°C) which is greater than 99% pure by HPLC analysis.

### Example 6

### Preparation of disodium 5-(methoxymethyl)pyridine-2,3-dicarboxylate from disodium [5,6-(dicarboxylate-3-pyridyl)methyl]trimethylammonium bromide (according to ex. 3 of EP-A 0 747 360)

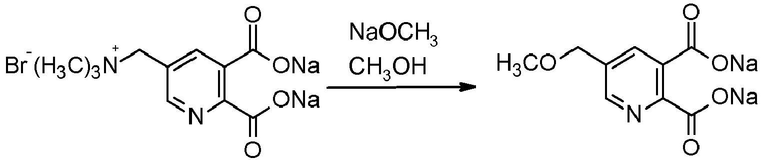

A mixture of disodium [(5,6-dicarboxylate-3-pyridyl)methyl]trimethylammonium bromide (5.0 g, 13.8 mmol) and a 25% wt/wt solution of sodium methoxide in methanol (4.46 g, 20.7 mmol of NaOCH₃) in 75 g of methanol is heated at 120°C for 21 hours in a pressure reactor. The reaction is cooled to room temperature, treated with water and concentrated to a final weight of 55.03 g. A 5.0 g sample is assayed by LC analysis (30% CH₃CN, 0.77 M H₃PO₄). The remainder of the reaction solution is evaporated to dryness to give a solid residue, identified by NMR analysis.

### Example 7

### Preparation of 5-(methoxymethyl)-2,3-pyridinedicarboxylic acid

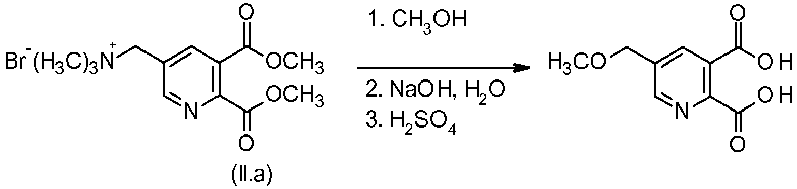

A ∼36% aqueous Quat Salt solution ([(5,6-dicarboxy-3-pyridyl)-methyl]trimethylammonium bromide, dimethyl ester (IIa)) (349 g, 0.365 mol) is concentrated to ∼64% by stripping H₂O by heating to 55°C and lowering pressure to 100 torr (199 g, 0.365 mol). After concentration, methanol is charged (418 g, 13.02 mol), then 50% NaOH is added over 30 minutes maintaining 45°C (166 g, 2.08 mol). Solution is transferred to a pressure reactor, sealed and heated to 80°C for 7 hours. Reaction temperature is then raised to 100°C and stirred for 4.5 hours. Temperature is lowered to 30°C, pressure released, and H₂O is added (183 g, 10.15 mol). Mixture is heated to 50°C and stirred for 30 minutes. Reaction solution is then heated to 100°C to strip off methanol to <1wt%, resulting is an AQ salt solution (430 g, 0.316 mol). To precipitate the desired isolated product, the AQ salt solution is heated to 56°C. H₂SO₄ is added to lower pH to 1.7 (84 g, 0.80 mol). The solids are filtered, washed with H₂O, and dried in vacuum oven.

## Claims

1. An improved process for manufacturing a compound of formula (I), 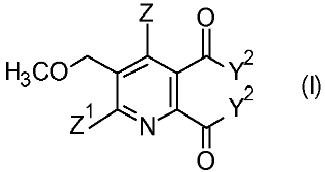 where
Z is hydrogen or halogen;
Z¹ is hydrogen, halogen, cyano or nitro;
Y² is OCH₃ or OM; and
M is an alkali metal;
comprising steps (ii) and (iii):
(ii) reacting a mixture of compounds of formulae (III.1), (III.2) and (III.3), 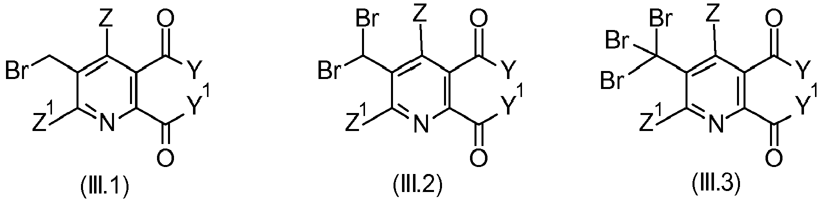 where
Z is hydrogen or halogen;
Z¹ is hydrogen, halogen, cyano or nitro;
Y, Y¹ are each independently OR¹, NR¹R², or when taken together YY¹ is -O-,-S- or -NR³-;
R¹, R² are each independently hydrogen, C₁-C₄ alkyl optionally substituted with C₁-C₄ alkoxy, or phenyl optionally substituted with one to three C₁-C₄ alkyl groups, C₁-C₄ alkoxy groups or halogen atoms;
R³ is hydrogen or C₁-C₄ alkyl;
with phosphorous acid (HPO(OH)₂) or a phosphite selected from dimethyl phosphite and diethyl phosphite, and trimethylamine in a solvent at a temperature range of 0°C to 100°C, to obtain a compound of formula (II) 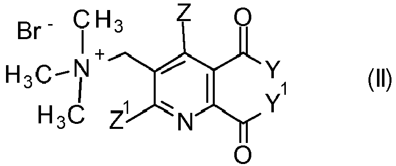 where Z, Z¹, Y, Y¹ are as defined in formulae (III.1), (III.2) and (III.3); and
(iii) reacting the compound of formula (II) in methanol, toluene or a methanol/toluene mixture with a base selected from MOCH₃ and MOH (if the solvent is methanol), where M is as defined in formula (I), to obtain the compound of formula (I).

2. The process as claimed in claim 1, where in step (ii) the phosphite is dimethyl phosphite.

3. The process as claimed in claim 1 or 2, where the mixture of compounds of formulae (III.1), (III.2) and (III.3) used in step (ii) is prepared in step (i) comprising:
(i) reacting a compound of formula (IV), 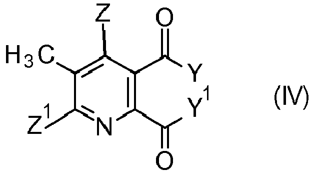 where
Z is hydrogen or halogen;
Z¹ is hydrogen, halogen, cyano or nitro;
Y, Y¹ are each independently OR¹, NR¹R², or when taken together YY¹ is -O-,-S- or -NR³-;
R¹, R² are each independently hydrogen, C₁-C₄ alkyl optionally substituted with C₁-C₄ alkoxy, or phenyl optionally substituted with one to three C₁-C₄ alkyl groups, C₁-C₄ alkoxy groups or halogen atoms;
R³ is hydrogen or C₁-C₄ alkyl;
with bromine in the presence of a radical initiator in a solvent mixture comprising an aqueous phase and an organic phase, where the organic phase comprises a solvent selected from 1,2-dichloroethane, chlorobenzene, 1,2-dichlorobenzene, 1,3-dichlorobenzene, 1,4-dichlorobenzene and tetrachloromethane, and where the pH-value of the aqueous phase is from 1 to < 8.

4. The process as claimed in claim 3, where the molar ratio of compound of formula (IV) to bromine in step (i) is 1:0.5-1.2.

5. The process as claimed in claims 3 or 4, where the radical initiator in step (i) is 2,2'-azobisisobutyronitrile.

6. The process as claimed in any one of claims 3 to 5, where the organic solvent in step (i) is 1,2-dichloroethane.

7. The process as claimed in any one of claims 3 to 6, where the pH-value of the aqueous phase in step (i) is from 1 to 8.

8. The process as claimed in any one of claims 3 to 7, where step (i) is carried out at a temperature of 50°C to 120°C.

9. The process as claimed in any one of claims 3 to 8, where the reaction in step (i) is carried out up to a conversion of compound (IV) of 20 to 60%.

10. The process as claimed in any one of claims 3 to 9, where the organic phase in step (ii) is recycled in step (i) with optional addition of compound (IV).

11. The process as claimed in any one of claims 3 to 10, where the organic phase in step (ii) is recycled in step (i) with addition of bromine and 2,2'-azobisisobutyronitrile.

12. An improved process for manufacturing a herbicidal imidazolinone compound of formula (V), 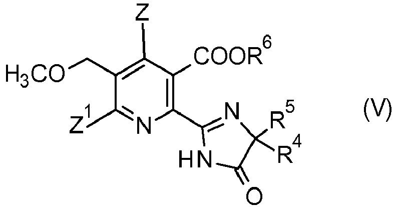 where
Z is hydrogen or halogen;
Z¹ is hydrogen, halogen, cyano or nitro;
R⁴ is C₁-C₄ alkyl;
R⁵ is C₁-C₄ alkyl, C₃-C₆ cycloalkyl, or R⁴ and R⁵, when taken together with the atom to which they are attached, represent a C₃-C₆ cycloalkyl group optionally substituted with methyl; and
R⁶ is hydrogen;
comprising the steps of:
preparing a compound having the formula (I) as described in any one of claims 1 to 11; and
(v) converting the compound of formula (I) into the herbicidal compound having formula (V).

13. The process as claimed in claim 12, comprising the steps of
preparing a compound having the formula (I), where Y² is OCH₃, as described in any one of claims 1 to 11;
(iv-1) optionally preparing the anhydride (VII) of compound (I); and
(iv-2) reacting compound (I) or its anhydride (VII) in the presence of a base with an 2-aminoalkane carboxamide of formula (VI),
H₂N-CR⁴R⁵-CONH₂ (VI)
where R⁴ and R⁵ are as in formula (V).

14. The process as claimed in claim 12, comprising the steps of
preparing a compound having the formula (I) as claimed in anyone of claims 1 to 11;
(iv-1) converting of compound (I) to the anhydride (VII); 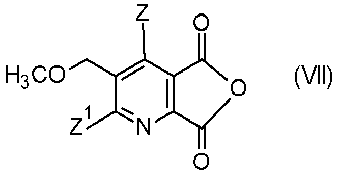
(iv-2) reacting anhydride (VII) with aminocarbonitrile (IX),
H₂N-CR⁴R⁵-CN (IX)
where R⁴ and R⁵ are as in formula (V),
to obtain amidonitrile compound (X), 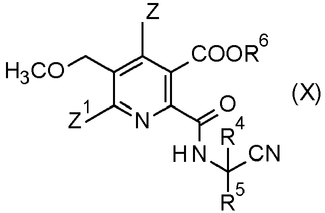 where the symbols are as in formula (V) in claim 12 and R⁶ is H;
(iv-3) hydrolyzing the nitrile group in compound (X) to yield amide (VIII), 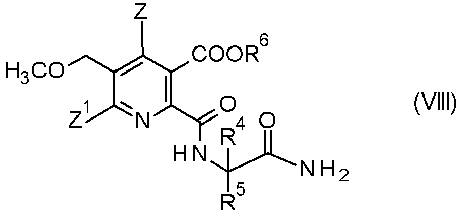 where the symbols are as in formula (V) in claim 12 and R⁶ is H, and
(iv-4) condensing amide (VIII) to yield the herbicidal imidazolinone (V).

15. The process as claimed in any one of claims 1 to 14, where in the compounds of formulae (III.1), (III.2) and (III.3)
Z is hydrogen;
Z¹ is hydrogen;
Y, Y¹ are each independently OR¹;
R¹ is hydrogen or methyl.
